# EUROPEAN PATENT APPLICATION

(11) **EP 4 527 454 A2**
(43) Date of publication of application: **26.03.2025**
(21) Application number: 24217104.9
(22) Date of filing: 05.03.2021
(51) Int. Cl.: A61P 3/00

(54) **METHODS OF TREATING FABRY DISEASE IN PATIENTS HAVING A MUTATION IN THE GLA GENE**

(30) Priority: 06.03.2020 US 202062986297 P
(62) Divisional of application: 21715035.8
(71) Applicant: Amicus Therapeutics, Inc., Philadelphia, PA 19104 (US)
(72) Inventor: BENJAMIN, Elfrida, Philadelphia, Pennsylvania, 19104 (US); WU, Xiaoyang, Philadelphia, Pennsylvania, 19104 (US)
(74) Representative: Hoffmann Eitle

(57) **Abstract**

Provided are methods of treating a patient diagnosed with Fabry disease and methods of enhancing α-galactosidase A in a patient diagnosed with or suspected of having Fabry disease. Certain methods comprise administering to a patient a therapeutically effective dose of a pharmacological chaperone for α-galactosidase A, wherein the patient has a mutation in the nucleic acid sequence encoding α-galactosidase A. Also described are uses of pharmacological chaperones for the treatment of Fabry disease and compositions for use in the treatment of Fabry disease.

## Description

### TECHNICAL FIELD

Principles and embodiments of the present invention relate generally to the use of pharmacological chaperones for the treatment of Fabry disease, particularly in patients with mutations or variants in the α-galactosidase (GLA) gene.

### BACKGROUND

Many human diseases result from mutations that cause changes in the amino acid sequence of a protein which reduce its stability and may prevent it from folding properly. Proteins generally fold in a specific region of the cell known as the endoplasmic reticulum, or ER. The cell has quality control mechanisms that ensure that proteins are folded into their correct three-dimensional shape before they can move from the ER to the appropriate destination in the cell, a process generally referred to as protein trafficking. Misfolded proteins are often eliminated by the quality control mechanisms after initially being retained in the ER. In certain instances, misfolded proteins can accumulate in the ER before being eliminated. The retention of misfolded proteins in the ER interrupts their proper trafficking, and the resulting reduced biological activity can lead to impaired cellular function and ultimately to disease. In addition, the accumulation of misfolded proteins in the ER may lead to various types of stress on cells, which may also contribute to cellular dysfunction and disease.

Such mutations can lead to lysosomal storage disorders (LSDs), which are characterized by deficiencies of lysosomal enzymes due to mutations in the genes encoding the lysosomal enzymes. The resultant disease causes the pathologic accumulation of substrates of those enzymes, which include lipids, carbohydrates, and polysaccharides. Although there are many different mutant genotypes associated with each LSD, many of the mutations are missense mutations which can lead to the production of a less stable enzyme. These less stable enzymes are sometimes prematurely degraded by the ER-associated degradation pathway. This results in the enzyme deficiency in the lysosome, and the pathologic accumulation of substrate. Such mutant enzymes are sometimes referred to in the pertinent art as "folding mutants" or "conformational mutants."

Fabry Disease is a LSD caused by a mutation to the GLA gene, which encodes the enzyme α-galactosidase A (α-Gal A). α-Gal A is required for glycosphingolipid metabolism. The mutation causes the substrate globotriaosylceramide (GL-3) to accumulate in various tissues and organs. Males with Fabry disease are hemizygotes because the disease genes are encoded on the X chromosome. Fabry disease is estimated to affect 1 in 40,000 and 60,000 males, and occurs less frequently in females.

There have been several approaches to treatment of Fabry disease. One approved therapy for treating Fabry disease is enzyme replacement therapy (ERT), which typically involves intravenous, infusion of a purified form of the corresponding wild-type protein. Two α-Gal A products are currently available for the treatment of Fabry disease: agalsidase alfa (Replagal^{®}, Shire Human Genetic Therapies) and agalsidase beta (Fabrazyme^{®}; Sanofi Genzyme Corporation). ERT has several drawbacks, however

r. One of the main complications with ERT is rapid degradation of the infused protein, which leads to the need for numerous costly high dose infusions. ERT has several additional caveats, such as difficulties with large-scale generation, purification, and storage of properly folded protein; obtaining glycosylated native protein; generation of an anti-protein immune response; and inability of protein to cross the blood-brain barrier to mitigate central nervous system pathologies (i.e., low bioavailability). In addition, replacement enzyme cannot penetrate the heart or kidney in sufficient amounts to reduce substrate accumulation in the renal podocytes or cardiac myocytes, which figure prominently in Fabry pathology.

Another approach to treating some enzyme deficiencies involves the use of small molecule inhibitors to reduce production of the natural substrate of deficient enzyme proteins, thereby ameliorating the pathology. This "substrate reduction" approach has been specifically described for a class of about 40 LSDs that include glycosphingolipid storage disorders. The small molecule inhibitors proposed for use as therapy are specific for inhibiting the enzymes involved in synthesis of glycolipids, reducing the amount of cellular glycolipid that needs to be broken down by the deficient enzyme.

A third approach to treating Fabry disease has been treatment with what are called pharmacological chaperones (PCs). Such PCs include small molecule inhibitors of α-Gal A, which can bind to the α-Gal A to increase the stability of both mutant enzyme and the corresponding wild type. However, patients for PC therapy should have an amenable mutation or variant which results in the production of an enzyme that has the potential to be stabilized and folded into a conformation that permits trafficking out of the ER.

Thus, even when Fabry disease is diagnosed by detecting deficient α-Gal A activity in plasma or peripheral leukocytes (WBCs), it is very difficult, if not impossible, to predict whether a particular Fabry patient will respond to treatment with a PC. Thus, there remains a need to identify new GLA mutations or variants that will be responsive to a PC and make available new methods of treatment to Fabry patients with these mutations or variants.

### SUMMARY

One aspect of the invention pertains to a method of treating a patient diagnosed with Fabry disease. The method comprises administering to the patient a therapeutically effective dose of a pharmacological chaperone for α-Gal A, wherein the patient has a missense mutation of the nucleic acid sequence encoding α-Gal A. In one or more embodiments, the mutation is A29D, R38S, N53Y, Y88C, V124G, I133F, A143V, Y152N, F159C, A160D, D165N, F169I, L180V, D182G, R196T, W209R, A257T, P259S, G271A, S276T, M290V, A291S, I303T, I303V, L310V, G360A, G360R, G375A, L394P, G411S, or N419D.

In various embodiments, these mutations are relative to SEQ ID NO: 2. In one or more embodiments, the mutation is A29D relative to SEQ ID NO: 2. In one or more embodiments, the mutation is R38S relative to SEQ ID NO: 2. In one or more embodiments, the mutation is N53Y relative to SEQ ID NO: 2. In one or more embodiments, the mutation is Y88C relative to SEQ ID NO: 2. In one or more embodiments, the mutation is V124G relative to SEQ ID NO: 2. In one or more embodiments, the mutation is I133F relative to SEQ ID NO: 2. In one or more embodiments, the mutation is A143V relative to SEQ ID NO: 2. In one or more embodiments, the mutation is Y152N relative to SEQ ID NO: 2. In one or more embodiments, the mutation is F159C relative to SEQ ID NO: 2. In one or more embodiments, the mutation is A160D relative to SEQ ID NO: 2. In one or more embodiments, the mutation is D165N relative to SEQ ID NO: 2. In one or more embodiments, the mutation is F169I relative to SEQ ID NO: 2. In one or more embodiments, the mutation is L180V relative to SEQ ID NO: 2. In one or more embodiments, the mutation is D182G relative to SEQ ID NO: 2. In one or more embodiments, the mutation is R196T relative to SEQ ID NO: 2. In one or more embodiments, the mutation is W209R relative to SEQ ID NO: 2. In one or more embodiments, the mutation is A257T relative to SEQ ID NO: 2. In one or more embodiments, the mutation is P259S relative to SEQ ID NO: 2. In one or more embodiments, the mutation is G271A relative to SEQ ID NO: 2. In one or more embodiments, the mutation is S276T relative to SEQ ID NO: 2. In one or more embodiments, the mutation is M290V relative to SEQ ID NO: 2. In one or more embodiments, the mutation is A291S relative to SEQ ID NO: 2. In one or more embodiments, the mutation is I303T relative to SEQ ID NO: 2. In one or more embodiments, the mutation is I303V relative to SEQ ID NO: 2. In one or more embodiments, the mutation is L310V relative to SEQ ID NO: 2. In one or more embodiments, the mutation is G360A relative to SEQ ID NO: 2. In one or more embodiments, the mutation is G360R relative to SEQ ID NO: 2. In one or more embodiments, the mutation is G375A relative to SEQ ID NO: 2. In one or more embodiments, the mutation is L394P relative to SEQ ID NO: 2. In one or more embodiments, the mutation is G411S relative to SEQ ID NO: 2. In one or more embodiments, the mutation is N419D relative to SEQ ID NO: 2.

In some embodiments, the pharmacological chaperone comprises migalastat or salt thereof. In one or more embodiments, the dose of migalastat or salt thereof is from about 100 mg to about 150 mg free base equivalent (FBE). In some embodiments, the salt of migalastat is migalastat hydrochloride. In one or more embodiments, the dose is about 150 mg every other day of migalastat hydrochloride or an equivalent dose of migalastat or a salt thereof other than the hydrochloride salt. In some embodiments, the migalastat or salt thereof is administered orally or by injection. These embodiments may be combined with one another or with other embodiments of the invention, for example embodiments relating to a method of enhancing α-Gal A in a patient diagnosed with or suspected of having Fabry disease, use of a pharmacological chaperone for α-Gal A for the manufacture of a medicament for treating a patient diagnosed with Fabry disease or to a pharmacological chaperone for α-Gal A for use in treating a patient diagnosed with Fabry disease as well as embodiments relating to amenable mutations, suitable PCs and dosages, formulations and routes of administration thereof.

Another aspect of the invention pertains to a method of enhancing α-Gal A in a patient diagnosed with or suspected of having Fabry disease. The method comprises administering to a patient a therapeutically effective dose of a pharmacological chaperone for α-Gal A, wherein the patient has a missense mutation in the nucleic acid sequence encoding α-Gal A. In one or more embodiments, the mutation is A29D, R38S, N53Y, Y88C, V124G, I133F, A143V, Y152N, F159C, A160D, D165N, F169I, L180V, D182G, R196T, W209R, A257T, P259S, G271A, S276T, M290V, A291S, I303T, I303V, L310V, G360A, G360R, G375A, L394P, G411S, or N419D.

In various embodiments, these mutations are relative to SEQ ID NO: 2. In one or more embodiments, the mutation is A29D relative to SEQ ID NO: 2. In one or more embodiments, the mutation is R38S relative to SEQ ID NO: 2. In one or more embodiments, the mutation is N53Y relative to SEQ ID NO: 2. In one or more embodiments, the mutation is Y88C relative to SEQ ID NO: 2. In one or more embodiments, the mutation is V124G relative to SEQ ID NO: 2. In one or more embodiments, the mutation is I133F relative to SEQ ID NO: 2. In one or more embodiments, the mutation is A143V relative to SEQ ID NO: 2. In one or more embodiments, the mutation is Y152N relative to SEQ ID NO: 2. In one or more embodiments, the mutation is F159C relative to SEQ ID NO: 2. In one or more embodiments, the mutation is A160D relative to SEQ ID NO: 2. In one or more embodiments, the mutation is D165N relative to SEQ ID NO: 2. In one or more embodiments, the mutation is F169I relative to SEQ ID NO: 2. In one or more embodiments, the mutation is L180V relative to SEQ ID NO: 2. In one or more embodiments, the mutation is D182G relative to SEQ ID NO: 2. In one or more embodiments, the mutation is R196T relative to SEQ ID NO: 2. In one or more embodiments, the mutation is W209R relative to SEQ ID NO: 2. In one or more embodiments, the mutation is A257T relative to SEQ ID NO: 2. In one or more embodiments, the mutation is P259S relative to SEQ ID NO: 2. In one or more embodiments, the mutation is G271A relative to SEQ ID NO: 2. In one or more embodiments, the mutation is S276T relative to SEQ ID NO: 2. In one or more embodiments, the mutation is M290V relative to SEQ ID NO: 2. In one or more embodiments, the mutation is A291S relative to SEQ ID NO: 2. In one or more embodiments, the mutation is I303T relative to SEQ ID NO: 2. In one or more embodiments, the mutation is I303V relative to SEQ ID NO: 2. In one or more embodiments, the mutation is L310V relative to SEQ ID NO: 2. In one or more embodiments, the mutation is G360A relative to SEQ ID NO: 2. In one or more embodiments, the mutation is G360R relative to SEQ ID NO: 2. In one or more embodiments, the mutation is G375A relative to SEQ ID NO: 2. In one or more embodiments, the mutation is L394P relative to SEQ ID NO: 2. In one or more embodiments, the mutation is G411S relative to SEQ ID NO: 2. In one or more embodiments, the mutation is N419D relative to SEQ ID NO: 2.

In some embodiments, the pharmacological chaperone comprises migalastat or salt thereof. In one or more embodiments, the dose of migalastat or salt thereof is from about 100 mg to about 150 mg FBE. In some embodiments, the salt of migalastat is migalastat hydrochloride. In one or more embodiments, the dose is about 150 mg every other day of migalastat hydrochloride or an equivalent dose of migalastat or a salt thereof other than the hydrochloride salt. In some embodiments, the migalastat or salt thereof is administered orally or by injection. These embodiments may be combined with one another or with other embodiments of the invention, for example embodiments relating to a method of treating a patient with Fabry disease, use of a pharmacological chaperone for α-Gal A for the manufacture of a medicament for treating a patient diagnosed with Fabry disease or to a pharmacological chaperone for α-Gal A for use in treating a patient diagnosed with Fabry disease as well as embodiments relating to amenable mutations, suitable PCs and dosages, formulations and routes of administration thereof.

Another aspect of the invention pertains to use of a pharmacological chaperone for α-Gal A for the manufacture of a medicament for treating a patient diagnosed with Fabry disease, wherein the patient has a missense mutation in the nucleic acid sequence encoding α-Gal A. In one or more embodiments, the mutation is A29D, R38S, N53Y, Y88C, V124G, I133F, A143V, Y152N, F159C, A160D, D165N, F169I, L180V, D182G, R196T, W209R, A257T, P259S, G271A, S276T, M290V, A291S, I303T, I303V, L310V, G360A, G360R, G375A, L394P, G411S, or N419D.

In various embodiments, these mutations are relative to SEQ ID NO: 2. In one or more embodiments, the mutation is A29D relative to SEQ ID NO: 2. In one or more embodiments, the mutation is R38S relative to SEQ ID NO: 2. In one or more embodiments, the mutation is N53Y relative to SEQ ID NO: 2. In one or more embodiments, the mutation is Y88C relative to SEQ ID NO: 2. In one or more embodiments, the mutation is V124G relative to SEQ ID NO: 2. In one or more embodiments, the mutation is I133F relative to SEQ ID NO: 2. In one or more embodiments, the mutation is A143V relative to SEQ ID NO: 2. In one or more embodiments, the mutation is Y152N relative to SEQ ID NO: 2. In one or more embodiments, the mutation is F159C relative to SEQ ID NO: 2. In one or more embodiments, the mutation is A160D relative to SEQ ID NO: 2. In one or more embodiments, the mutation is D165N relative to SEQ ID NO: 2. In one or more embodiments, the mutation is F169I relative to SEQ ID NO: 2. In one or more embodiments, the mutation is L180V relative to SEQ ID NO: 2. In one or more embodiments, the mutation is D182G relative to SEQ ID NO: 2. In one or more embodiments, the mutation is R196T relative to SEQ ID NO: 2. In one or more embodiments, the mutation is W209R relative to SEQ ID NO: 2. In one or more embodiments, the mutation is A257T relative to SEQ ID NO: 2. In one or more embodiments, the mutation is P259S relative to SEQ ID NO: 2. In one or more embodiments, the mutation is G271A relative to SEQ ID NO: 2. In one or more embodiments, the mutation is S276T relative to SEQ ID NO: 2. In one or more embodiments, the mutation is M290V relative to SEQ ID NO: 2. In one or more embodiments, the mutation is A291S relative to SEQ ID NO: 2. In one or more embodiments, the mutation is I303T relative to SEQ ID NO: 2. In one or more embodiments, the mutation is I303V relative to SEQ ID NO: 2. In one or more embodiments, the mutation is L310V relative to SEQ ID NO: 2. In one or more embodiments, the mutation is G360A relative to SEQ ID NO: 2. In one or more embodiments, the mutation is G360R relative to SEQ ID NO: 2. In one or more embodiments, the mutation is G375A relative to SEQ ID NO: 2. In one or more embodiments, the mutation is L394P relative to SEQ ID NO: 2. In one or more embodiments, the mutation is G411S relative to SEQ ID NO: 2. In one or more embodiments, the mutation is N419D relative to SEQ ID NO: 2.

In some embodiments, the pharmacological chaperone comprises migalastat or salt thereof. In one or more embodiments, the dose of migalastat or salt thereof is from about 100 mg to about 150 mg FBE. In some embodiments, the salt of migalastat is migalastat hydrochloride. In one or more embodiments, the dose is about 150 mg every other day of migalastat hydrochloride or an equivalent dose of migalastat or a salt thereof other than the hydrochloride salt. In some embodiments, the migalastat or salt thereof is administered orally or by injection. These embodiments may be combined with one another or with other embodiments of the invention, for example embodiments relating to a method of treating a patient with Fabry disease, a method of enhancing α-Gal A in a patient diagnosed with or suspected of having Fabry disease, or to a pharmacological chaperone for α-Gal A for use in treating a patient diagnosed with Fabry disease as well as embodiments relating to amenable mutations, suitable PCs and dosages, formulations and routes of administration thereof.

Another aspect of the invention pertains to a pharmacological chaperone for α-Gal A for use in treating a patient diagnosed with Fabry disease, wherein the patient has a missense mutation in the nucleic acid sequence encoding α-Gal A. In one or more embodiments, the mutation is A29D, R38S, N53Y, Y88C, V124G, I133F, A143V, Y152N, F159C, A160D, D165N, F169I, L180V, D182G, R196T, W209R, A257T, P259S, G271A, S276T, M290V, A291S, I303T, I303V, L310V, G360A, G360R, G375A, L394P, G411S, or N419D.

In various embodiments, these mutations are relative to SEQ ID NO: 2. In one or more embodiments, the mutation is A29D relative to SEQ ID NO: 2. In one or more embodiments, the mutation is R38S relative to SEQ ID NO: 2. In one or more embodiments, the mutation is N53Y relative to SEQ ID NO: 2. In one or more embodiments, the mutation is Y88C relative to SEQ ID NO: 2. In one or more embodiments, the mutation is V124G relative to SEQ ID NO: 2. In one or more embodiments, the mutation is I133F relative to SEQ ID NO: 2. In one or more embodiments, the mutation is A143V relative to SEQ ID NO: 2. In one or more embodiments, the mutation is Y152N relative to SEQ ID NO: 2. In one or more embodiments, the mutation is F159C relative to SEQ ID NO: 2. In one or more embodiments, the mutation is A160D relative to SEQ ID NO: 2. In one or more embodiments, the mutation is D165N relative to SEQ ID NO: 2. In one or more embodiments, the mutation is F169I relative to SEQ ID NO: 2. In one or more embodiments, the mutation is L180V relative to SEQ ID NO: 2. In one or more embodiments, the mutation is D182G relative to SEQ ID NO: 2. In one or more embodiments, the mutation is R196T relative to SEQ ID NO: 2. In one or more embodiments, the mutation is W209R relative to SEQ ID NO: 2. In one or more embodiments, the mutation is A257T relative to SEQ ID NO: 2. In one or more embodiments, the mutation is P259S relative to SEQ ID NO: 2. In one or more embodiments, the mutation is G271A relative to SEQ ID NO: 2. In one or more embodiments, the mutation is S276T relative to SEQ ID NO: 2. In one or more embodiments, the mutation is M290V relative to SEQ ID NO: 2. In one or more embodiments, the mutation is A291S relative to SEQ ID NO: 2. In one or more embodiments, the mutation is I303T relative to SEQ ID NO: 2. In one or more embodiments, the mutation is I303V relative to SEQ ID NO: 2. In one or more embodiments, the mutation is L310V relative to SEQ ID NO: 2. In one or more embodiments, the mutation is G360A relative to SEQ ID NO: 2. In one or more embodiments, the mutation is G360R relative to SEQ ID NO: 2. In one or more embodiments, the mutation is G375A relative to SEQ ID NO: 2. In one or more embodiments, the mutation is L394P relative to SEQ ID NO: 2. In one or more embodiments, the mutation is G411S relative to SEQ ID NO: 2. In one or more embodiments, the mutation is N419D relative to SEQ ID NO: 2.

In some embodiments, the pharmacological chaperone comprises migalastat or salt thereof. In one or more embodiments, the dose of migalastat or salt thereof is from about 100 mg to about 150 mg FBE. In some embodiments, the salt of migalastat is migalastat hydrochloride. In one or more embodiments, the dose is about 150 mg every other day of migalastat hydrochloride or an equivalent dose of migalastat or a salt thereof other than the hydrochloride salt. In some embodiments, the migalastat or salt thereof is administered orally or by injection. These embodiments may be combined with one another or with other embodiments of the invention, for example embodiments relating to a method of treating a patient with Fabry disease, a method of enhancing α-Gal A in a patient diagnosed with or suspected of having Fabry disease or use of a pharmacological chaperone for α-Gal A for the manufacture of a medicament for treating a patient diagnosed with Fabry disease as well as embodiments relating to amenable mutations, suitable PCs and dosages, formulations and routs of administration thereof.

Various embodiments are listed below. It will be understood that the embodiments listed below may be combined not only as listed below, but in other suitable combinations in accordance with the scope of the invention.

### BRIEF DESCRIPTION OF THE DRAWINGS

FIGS. 1A-E shows the full DNA sequence of human wild type GLA gene (SEQ ID NO: 1);
FIG. 2 shows the wild-type α-Gal A protein (SEQ ID NO: 2); and
FIG. 3 shows the nucleic acid sequence encoding the wild-type α-Gal A protein (SEQ ID NO: 3).

### DETAILED DESCRIPTION

Before describing several exemplary embodiments of the invention, it is to be understood that the invention is not limited to the details of construction or process steps set forth in the following description. The invention is capable of other embodiments and of being practiced or being carried out in various ways.

Various aspects of the invention pertain to identification of new GLA mutations in Fabry patients who will respond to treatment with pharmacological chaperones. Other aspects of the invention pertain to the treatment of these Fabry patients, as well. For example, it has been unexpectedly discovered that the low α-Gal A activity resulting from the missense mutations A29D, R38S, N53Y, Y88C, V124G, I133F, A143V, Y152N, F159C, A160D, D165N, F169I, L180V, D182G, R196T, W209R, A257T, P259S, G271A, S276T, M290V, A291S, I303T, I303V, L310V, G360A, G360R, G375A, L394P, G411S, or N419D in α-Gal A can be increased when exposed to pharmacological chaperones. By extension, patients with these mutations will be responsive to treatment with pharmacological chaperones.

### Definitions

The terms used in this specification generally have their ordinary meanings in the art, within the context of this invention and in the specific context where each term is used. Certain terms are discussed below, or elsewhere in the specification, to provide additional guidance to the practitioner in describing the compositions and methods of the invention and how to make and use them.

The term "Fabry disease" refers to an X-linked inborn error of glycosphingolipid catabolism due to deficient lysosomal α-Gal A activity. This defect causes accumulation of the substrate globotriaosylceramide (("GL-3", also known as Gb₃ or ceramide trihexoside) and related glycosphingolipids in vascular endothelial lysosomes of the heart, kidneys, skin, and other tissues. Another substrate of the enzyme is plasma globotriaosylsphingosine ("plasma lyso-Gbs").

A "carrier" is a female who has one X chromosome with a defective α-Gal A gene and one X chromosome with the normal gene and in whom X chromosome inactivation of the normal allele is present in one or more cell types. A carrier is often diagnosed with Fabry disease.

A "patient" refers to a subject who has been diagnosed with or is suspected of having a particular disease. The patient may be human or animal.

A "Fabry patient" refers to an individual who has been diagnosed with or suspected of having Fabry disease and has a mutated α-Gal A as defined further below. Characteristic markers of Fabry disease can occur in male hemizygotes and female carriers with the same prevalence, although females typically are less severely affected.

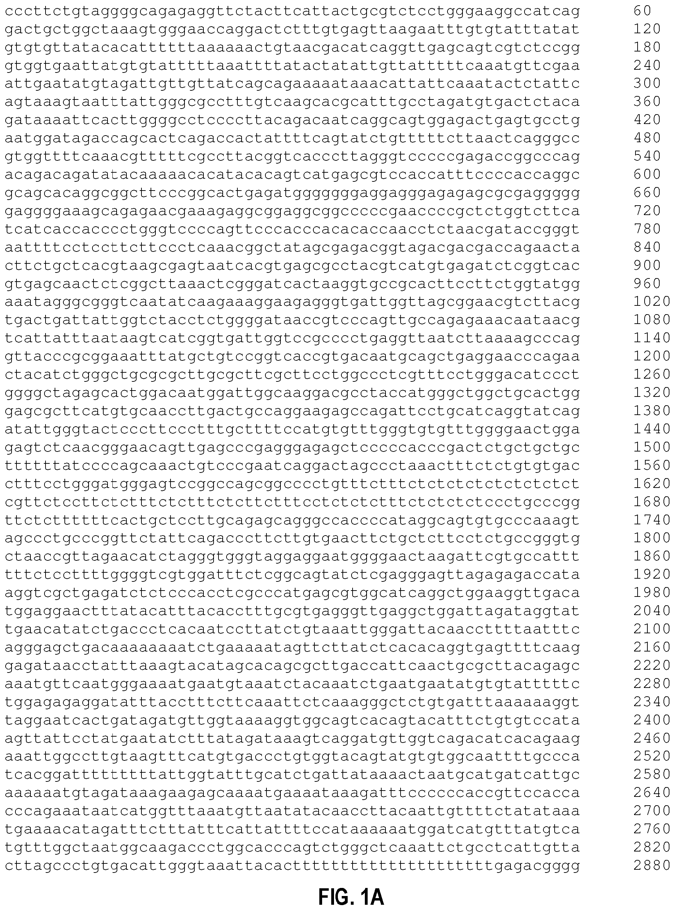

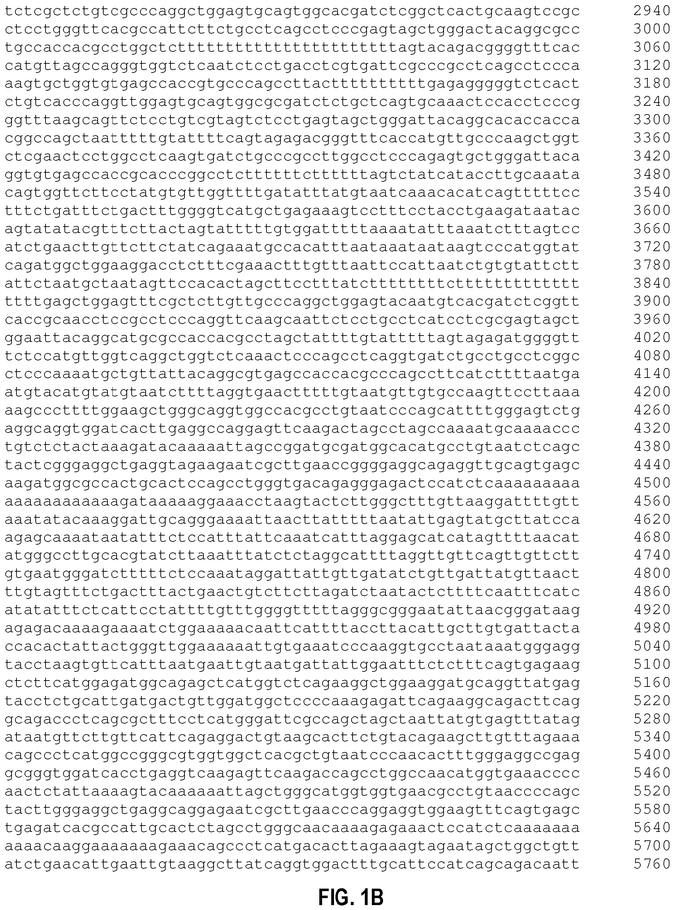

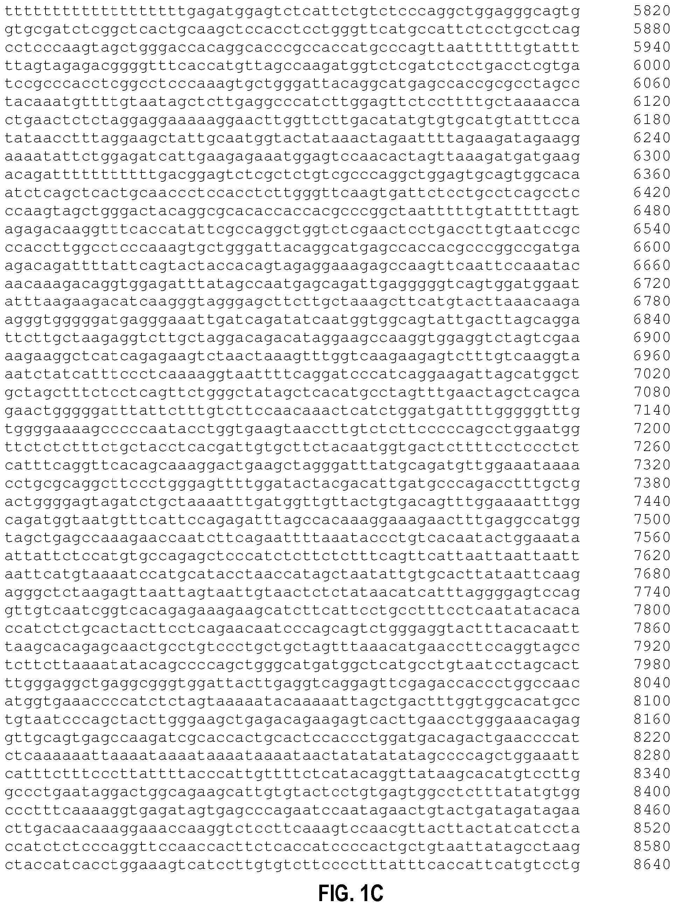

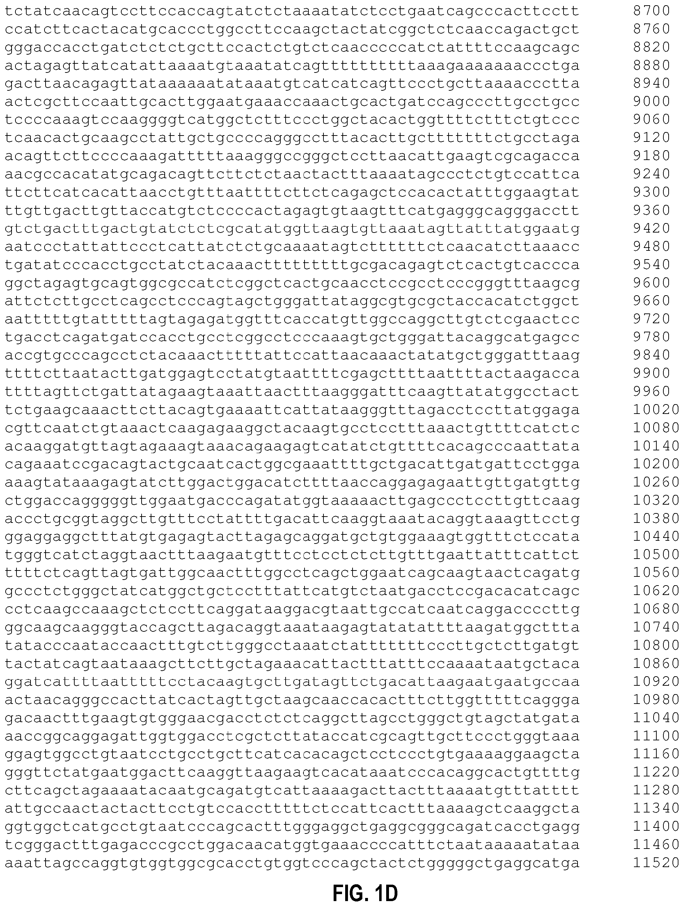

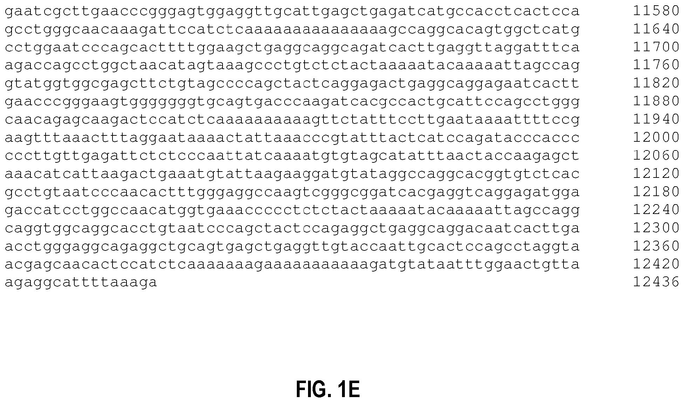

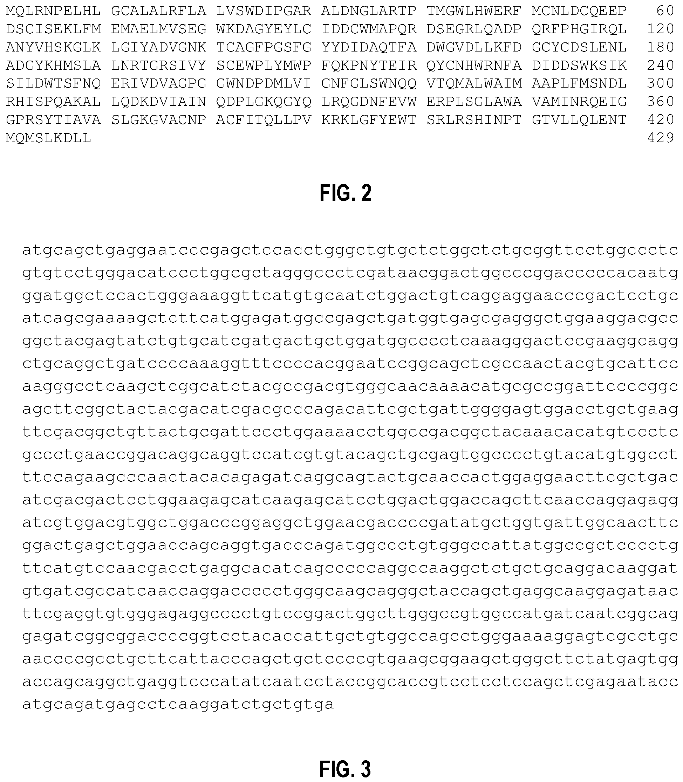

Human α-galactosidase A (α-Gal A) refers to an enzyme encoded by the human GLA gene. The full DNA sequence of α-Gal A, including introns and exons, is available in GenBank Accession No. X14448.1 and shown in FIGS. 1A-E (SEQ ID NO: 1). The human α-Gal A enzyme consists of 429 amino acids and is available in GenBank Accession Nos. X14448.1 and U78027 and shown in FIG. 2 (SEQ ID NO: 2). The nucleic acid sequence that only includes the coding regions (i.e. exons) of SEQ ID NO: 1 is shown in FIG. 3 (SEQ ID NO: 3).

The term "mutant protein" includes a protein which has a mutation in the gene encoding the protein which results in the inability of the protein to achieve a stable conformation under the conditions normally present in the ER. The failure to achieve a stable conformation results in a substantial amount of the enzyme being degraded, rather than being transported to the lysosome. Such a mutation is sometimes called a "conformational mutant." Such mutations include, but are not limited to, missense mutations, and in-frame small deletions and insertions. As used herein, deletions are indicated by the abbreviation "del" and insertions are indicated by the abbreviation "ins". Thus, the nucleotide change "c.184_185insTAG" refers to an insertion of the nucleotide sequence TAG between nucleotides 184 and 185 and the protein sequence change "S62delinsLA" refers to a deletion of the amino acid S (serine) at position 62 and an insertion of the amino acid sequence LA (leucine and alanine).

As used herein in one embodiment, the term "mutant α-Gal A" includes an α-Gal A which has a mutation in the gene encoding α-Gal A which results in the inability of the enzyme to achieve a stable conformation under the conditions normally present in the ER. The failure to achieve a stable conformation results in a substantial amount of the enzyme being degraded, rather than being transported to the lysosome.

As used herein, the term "specific pharmacological chaperone" ("SPC") or "pharmacological chaperone" ("PC") refers to any molecule including a small molecule, protein, peptide, nucleic acid, carbohydrate, etc. that specifically binds to a protein and has one or more of the following effects: (i) enhances the formation of a stable molecular conformation of the protein; (ii) induces trafficking of the protein from the ER to another cellular location, preferably a native cellular location, i.e., prevents ER-associated degradation of the protein; (iii) prevents aggregation of misfolded proteins; and/or (iv) restores or enhances at least partial wild-type function and/or activity to the protein. A compound that specifically binds to e.g., α-Gal A, means that it binds to and exerts a chaperone effect on the enzyme and not a generic group of related or unrelated enzymes. More specifically, this term does not refer to endogenous chaperones, such as BiP, or to non-specific agents which have demonstrated non-specific chaperone activity against various proteins, such as glycerol, DMSO or deuterated water, i.e., chemical chaperones. In one or more embodiments of the present invention, the PC may be a reversible competitive inhibitor. In one embodiment, the PC is migalastat or a salt thereof. In another embodiment, the PC is migalastat free base (e.g., 123 mg of migalastat free base). In yet another embodiment, the PC is a salt of migalastat (e.g., 150 mg of migalastat HCl).

A "competitive inhibitor" of an enzyme can refer to a compound which structurally resembles the chemical structure and molecular geometry of the enzyme substrate to bind the enzyme in approximately the same location as the substrate. Thus, the inhibitor competes for the same active site as the substrate molecule, thus increasing the Km. Competitive inhibition is usually reversible if sufficient substrate molecules are available to displace the inhibitor, i.e., competitive inhibitors can bind reversibly. Therefore, the amount of enzyme inhibition depends upon the inhibitor concentration, substrate concentration, and the relative affinities of the inhibitor and substrate for the active site.

As used herein, the term "specifically binds" refers to the interaction of a pharmacological chaperone with a protein such as α-Gal A, specifically, an interaction with amino acid residues of the protein that directly participate in contacting the pharmacological chaperone. A pharmacological chaperone specifically binds a target protein, e.g., α-Gal A, to exert a chaperone effect on the protein and not a generic group of related or unrelated proteins. The amino acid residues of a protein that interact with any given pharmacological chaperone may or may not be within the protein's "active site." Specific binding can be evaluated through routine binding assays or through structural studies, e.g., co-crystallization, NMR, and the like. The active site for α-Gal A is the substrate binding site.

"Deficient α-Gal A activity" refers to α-Gal A activity in cells from a patient which is below the normal range as compared (using the same methods) to the activity in normal individuals not having or suspected of having Fabry or any other disease (especially a blood disease).

As used herein, the terms "enhance α-Gal A activity" or "increase α-Gal A activity" refer to increasing the amount of α-Gal A that adopts a stable conformation in a cell contacted with a pharmacological chaperone specific for the α-Gal A, relative to the amount in a cell (preferably of the same cell-type or the same cell, e.g., at an earlier time) not contacted with the pharmacological chaperone specific for the α-Gal A. This term also refers to increasing the trafficking of α-Gal A to the lysosome in a cell contacted with a pharmacological chaperone specific for the α-Gal A, relative to the trafficking of α-Gal A not contacted with the pharmacological chaperone specific for the protein. These terms refer to both wild-type and mutant α-Gal A. In one embodiment, the increase in the amount of α-Gal A in the cell is measured by measuring the hydrolysis of an artificial substrate in lysates from cells that have been treated with the PC. An increase in hydrolysis is indicative of increased α-Gal A activity.

The term "α-Gal A activity" refers to the normal physiological function of a wild-type α-Gal A in a cell. For example, α-Gal A activity includes hydrolysis of GL-3.

A "responder" is an individual diagnosed with or suspected of having a lysosomal storage disorder, such, for example Fabry disease, whose cells exhibit sufficiently increased α-Gal A activity, respectively, and/or amelioration of symptoms or enhancement in surrogate markers, in response to contact with a PC. Non-limiting examples of enhancements in surrogate markers for Fabry are lyso-GB₃ and those disclosed in US Patent Application Publication No. US 2010-0113517, which is hereby incorporated by reference in its entirety.

Non-limiting examples of improvements in surrogate markers for Fabry disease disclosed in US 2010/0113517 include increases in α-Gal A levels or activity in cells (*e.g.,* fibroblasts) and tissue; reductions in of GL-3 accumulation; decreased plasma concentrations of homocysteine and vascular cell adhesion molecule-1 (VCAM-1); decreased GL-3 accumulation within myocardial cells and valvular fibrocytes; reduction in plasma lyso-Gb₃; reduction in cardiac hypertrophy (especially of the left ventricle), amelioration of valvular insufficiency, and arrhythmias; amelioration of proteinuria; decreased urinary concentrations of lipids such as CTH, lactosylceramide, ceramide, and increased urinary concentrations of glucosylceramide and sphingomyelin; the absence of laminated inclusion bodies (Zebra bodies) in glomerular epithelial cells; improvements in renal function; mitigation of hypohidrosis; the absence of angiokeratomas; and improvements in hearing abnormalities such as high frequency sensorineural hearing loss progressive hearing loss, sudden deafness, or tinnitus. Improvements in neurological symptoms include prevention of transient ischemic attack (TIA) or stroke; and amelioration of neuropathic pain manifesting itself as acroparaesthesia (burning or tingling in extremities). Another type of clinical marker that can be assessed for Fabry disease is the prevalence of deleterious cardiovascular manifestations. Common cardiac-related signs and symptoms of Fabry disease include left ventricular hypertrophy, valvular disease (especially mitral valve prolapse and/or regurgitation), premature coronary artery disease, angina, myocardial infarction, conduction abnormalities, arrhythmias, congestive heart failure.

The dose that achieves one or more of the aforementioned responses is a "therapeutically effective dose."

The phrase "pharmaceutically acceptable" refers to molecular entities and compositions that are physiologically tolerable and do not typically produce untoward reactions when administered to a human. In some embodiments, as used herein, the term "pharmaceutically acceptable" means approved by a regulatory agency of the Federal or a state government or listed in the U.S. Pharmacopoeia or other generally recognized pharmacopoeia for use in animals, and more particularly in humans. The term "carrier" in reference to a pharmaceutical carrier refers to a diluent, adjuvant, excipient, or vehicle with which the compound is administered. Such pharmaceutical carriers can be sterile liquids, such as water and oils. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin, 18th Edition, or other editions.

As used herein, the term "isolated" means that the referenced material is removed from the environment in which it is normally found. Thus, an isolated biological material can be free of cellular components, i.e., components of the cells in which the material is found or produced. In the case of nucleic acid molecules, an isolated nucleic acid includes a PCR product, an mRNA band on a gel, a cDNA, or a restriction fragment. In another embodiment, an isolated nucleic acid is preferably excised from the chromosome in which it may be found, and more preferably is no longer joined to non-regulatory, non-coding regions, or to other genes, located upstream or downstream of the gene contained by the isolated nucleic acid molecule when found in the chromosome. In yet another embodiment, the isolated nucleic acid lacks one or more introns. Isolated nucleic acids include sequences inserted into plasmids, cosmids, artificial chromosomes, and the like. Thus, in a specific embodiment, a recombinant nucleic acid is an isolated nucleic acid. An isolated protein may be associated with other proteins or nucleic acids, or both, with which it associates in the cell, or with cellular membranes if it is a membrane-associated protein. An isolated organelle, cell, or tissue is removed from the anatomical site in which it is found in an organism. An isolated material may be, but need not be, purified.

The terms "about" and "approximately" shall generally mean an acceptable degree of error for the quantity measured given the nature or precision of the measurements. Typical, exemplary degrees of error are within 20 percent (%), preferably within 10%, and more preferably within 5% of a given value or range of values. Alternatively, and particularly in biological systems, the terms "about" and "approximately" may mean values that are within an order of magnitude, preferably within 10- or 5-fold, and more preferably within 2-fold of a given value. Numerical quantities given herein are approximate unless stated otherwise, meaning that the term "about" or "approximately" can be inferred when not expressly stated.

The term "enzyme replacement therapy" or "ERT" refers to the introduction of a non-native, purified enzyme into an individual having a deficiency in such enzyme. The administered protein can be obtained from natural sources or by recombinant expression (as described in greater detail below). The term also refers to the introduction of a purified enzyme in an individual otherwise requiring or benefiting from administration of a purified enzyme, e.g., suffering from enzyme insufficiency. The introduced enzyme may be a purified, recombinant enzyme produced *in vitro,* or protein purified from isolated tissue or fluid, such as, e.g., placenta or animal milk, or from plants.

As used herein, the term "free base equivalent" or "FBE" refers to the amount of migalastat present in the migalastat or salt thereof. In other words, the term "FBE" means either an amount of migalastat free base, or the equivalent amount of migalastat free base that is provided by a salt of migalastat. For example, due to the weight of the hydrochloride salt, 150 mg of migalastat hydrochloride only provides as much migalastat as 123 mg of the free base form of migalastat. Other salts are expected to have different conversion factors, depending on the molecular weight of the salt.

The term "migalastat" encompasses migalastat free base or a pharmaceutically acceptable salt thereof (e.g., migalastat HCl), unless specifically indicated to the contrary.

The terms "mutation" and "variant" (e.g., as in "amenable mutation or variant") refer to a change in the nucleotide sequence of a gene or a chromosome. The two terms referred herein are typically used together - e.g., as in "mutation or variant"- referring to the change in nucleotide sequence stated in the previous sentence. If only one of the two terms is recited for some reason, the missing term was intended to be included and one should understand as such. Furthermore, the terms "amenable mutation" and "amenable variant" refer to a mutation or variant that is amenable to PC therapy, e.g. a mutation that is amenable to migalastat therapy. A particular type of amenable mutation or variant is a "HEK assay amenable mutation or variant", which is a mutation or variant that is determined to be amenable to migalastat therapy according to the criteria in the *in vitro* HEK assay described herein.

### Fabry Disease

Fabry disease is a rare, progressive and devastating X-linked lysosomal storage disorder. Mutations in the GLA gene result in a deficiency of the lysosomal enzyme, α-Gal A, which is required for glycosphingolipid metabolism. Beginning early in life, the reduction in α-Gal A activity results in an accumulation of glycosphingolipids, including GL-3 and plasma lyso-Gb₃, and leads to the symptoms and life-limiting sequelae of Fabry disease, including pain, gastrointestinal symptoms, renal failure, cardiomyopathy, cerebrovascular events, and early mortality. Early initiation of therapy and lifelong treatment provide an opportunity to slow disease progression and prolong life expectancy.

Fabry disease encompasses a spectrum of disease severity and age of onset, although it has traditionally been divided into 2 main phenotypes, "classic" and "late-onset". The classic phenotype has been ascribed primarily to males with undetectable to low α-Gal A activity and earlier onset of renal, cardiac and/or cerebrovascular manifestations. The late-onset phenotype has been ascribed primarily to males with higher residual α-Gal A activity and later onset of these disease manifestations. Heterozygous female carriers typically express the late-onset phenotype but depending on the pattern of X-chromosome inactivation may also display the classic phenotype.

More than 1,000 Fabry disease-causing GLA mutations have been identified. Approximately 60% are missense mutations, resulting in single amino acid substitutions in the α-Gal A enzyme. Missense GLA mutations often result in the production of abnormally folded and unstable forms of α-Gal A and the majority are associated with the classic phenotype. Normal cellular quality control mechanisms in the endoplasmic reticulum block the transit of these abnormal proteins to lysosomes and target them for premature degradation and elimination. Many missense mutant forms are targets for migalastat, an α-Gal A-specific pharmacological chaperone.

The clinical manifestations of Fabry disease span a broad spectrum of severity and roughly correlate with a patient's residual α-GAL levels. The majority of currently treated patients are referred to as classic Fabry disease patients, most of whom are males. These patients experience disease of various organs, including the kidneys, heart and brain, with disease symptoms first appearing in adolescence and typically progressing in severity until death in the fourth or fifth decade of life. A number of recent studies suggest that there are a large number of undiagnosed males and females that have a range of Fabry disease symptoms, such as impaired cardiac or renal function and strokes, that usually first appear in adulthood. Individuals with this type of Fabry disease, referred to as late-onset Fabry disease, tend to have higher residual α-GAL levels than classic Fabry disease patients. Individuals with late-onset Fabry disease typically first experience disease symptoms in adulthood, and often have disease symptoms focused on a single organ, such as enlargement of the left ventricle or progressive kidney failure. In addition, late-onset Fabry disease may also present in the form of strokes of unknown cause.

Fabry patients have progressive kidney impairment, and untreated patients exhibit end-stage renal impairment by the fifth decade of life. Deficiency in α-Gal A activity leads to accumulation of GL-3 and related glycosphingolipids in many cell types including cells in the kidney. GL-3 accumulates in podocytes, epithelial cells and the tubular cells of the distal tubule and loop of Henle. Impairment in kidney function can manifest as proteinuria and reduced glomerular filtration rate.

Because Fabry disease is rare, involves multiple organs, has a wide age range of onset, and is heterogeneous, proper diagnosis is a challenge. Awareness is low among health care professionals and misdiagnoses are frequent. Diagnosis of Fabry disease is most often confirmed on the basis of decreased α-Gal A activity in plasma or peripheral leukocytes (WBCs) once a patient is symptomatic, coupled with mutational analysis. In females, diagnosis is even more challenging since the enzymatic identification of carrier females is less reliable due to random X-chromosomal inactivation in some cells of carriers. For example, some obligate carriers (daughters of classically affected males) have α-Gal A enzyme activities ranging from normal to very low activities. Since carriers can have normal α-Gal A enzyme activity in leukocytes, only the identification of an α-Gal A mutation by genetic testing provides precise carrier identification and/or diagnosis.

Also, as described above, the age of onset, progression, and severity of Fabry disease is at least partly dependent on the rate of substrate accumulation, which correlates to the enzymatic activity in lysosomes. Thus, a complete lack of residual activity can correspond to rapid substrate accumulation, and therefore a more severe form of the disease (having early onset and rapid progression). However, even small quantities of residual activity may be enough to degrade a large amounts of substrate. This in turn would lead to milder disease with later onset and slower progression because of the slowed substrate accumulation. Considering these factors, it is thought that even modest increases in enzymatic activity can reduce the effect of a severe clinical phenotype. Data suggests that for most LSDs, just 1% to 6% of normal activity has been estimated as sufficient to delay or prevent disease onset or yield a more mild form of the disease. That is, just small increases in activity could have a significant impact on substrate levels, and hence disease severity and the rate of disease progression. Conversely, it is expected that a mutant lysosomal enzyme that shows no response *in vitro* would also not respond *in vivo.*

In one or more embodiments, mutant or variant forms of α-Gal A considered to be amenable to migalastat are defined as showing a relative increase (+10 µM migalastat) of ≥1.20-fold and an absolute increase (+ 10 µM migalastat) of ≥ 3.0% wild-type when the mutant form of α-Gal A is expressed in HEK-293 cells (referred to as the "HEK assay") according to Good Laboratory Practice (GLP)-validated *in vitro* assay (GLP HEK or Migalastat Amenability Assay). Such mutations or variants are also referred to herein as "HEK assay amenable" mutations or variants.

Previous screening methods have been provided that assess enzyme enhancement prior to the initiation of treatment. For example, an assay using HEK-293 cells has been utilized in clinical trials to predict whether a given mutation will be responsive to pharmacological chaperone (*e.g*., migalastat) treatment. In this assay, cDNA constructs are created. The corresponding α-Gal A mutant forms are transiently expressed in HEK-293 cells. Cells are then incubated ± migalastat (17 nM to 1 mM) for 4 to 5 days. After, α-Gal A levels are measured in cell lysates using a synthetic fluorogenic substrate (4-MU-α-Gal) or by western blot. This has been done for known disease-causing missense or small in-frame insertion/deletion mutations. Mutations that have previously been identified as responsive to a PC (*e.g.* migalastat) using these methods are listed in U.S. Pat. No. 8,592,362, which is hereby incorporated by reference in its entirety.

HEK assay amenable mutations include at least those mutations listed in a pharmacological reference table (e.g., the ones recited in the U.S. or International Product labels for a migalastat product such as GALAFOLD^{®}). As used herein, "pharmacological reference table" refers to any publicly accessible written or electronic record, included in either the product label within the packaging of a migalastat product (e.g., GALAFOLD^{®}) or in a website accessible by health care providers, that conveys whether a particular mutation or variant is responsive to migalastat (e.g., GALAFOLD^{®}) PC therapy, and is not necessarily limited to written records presented in tabular form. In one embodiment of the present invention, a "pharmacological reference table" thus refers to any depository of information that includes one or more amenable mutations or variants. In another embodiment, a "pharmacological reference table" refers to an updated depository of amenable mutations or variants that includes the novel mutations or variants disclosed herein (i.e.,A29D, R38S, N53Y, Y88C, V124G, I133F, A143V, Y152N, F159C, A160D, D165N, F169I, L180V, D182G, R196T, W209R, A257T, P259S, G271A, S276T, M290V, A291S, I303T, I303V, L310V, G360A, G360R, G375A, L394P, G411S, or N419D). An exemplary pharmacological reference table for HEK assay amenable mutations can be found in the summary of product characteristics and/or prescribing information for GALAFOLD^{®} in various countries in which GALAFOLD^{®} is approved for use, or at a website such as www.galafoldamenabilitytable.com or www.fabrygenevariantsearch.com, each of which is hereby incorporated by reference in its entirety.

However, as only certain mutations are amenable to treatment with migalastat, there is a need to identify new mutations and determine whether such mutations are amenable to migalastat therapy. As described in the Example below, several new mutations have been identified and determined to be mutations that are amenable to migalastat therapy. These mutations include A29D, R38S, N53Y, Y88C, V124G, I133F, A143V, Y152N, F159C, A160D, D165N, F169I, L180V, D182G, R196T, W209R, A257T, P259S, G271A, S276T, M290V, A291S, I303T, I303V, L310V, G360A, G360R, G375A, L394P, G411S, and N419D.

Accordingly, in one or more embodiments, migalastat is used to treat Fabry disease and/or enhance α-Gal A activity in a patient having an α-Gal A mutation selected from the group consisting of: A29D, R38S, N53Y, Y88C, V124G, I133F, A143V, Y152N, F159C, A160D, and D165N. In one or more embodiments, migalastat is used to treat Fabry disease and/or enhance α-Gal A activity in a patient having an α-Gal A mutation selected from the group consisting of: F169I, L180V, D182G, R196T, W209R, A257T, P259S, G271A, S276T, and M290V. In one or more embodiments, migalastat is used to treat Fabry disease and/or enhance α-Gal A activity in a patient having an α-Gal A mutation selected from the group consisting of: A291S, I303T, I303V, L310V, G360A, G360R, G375A, L394P, G411S, and N419D.

In one or more embodiments, the patient has the mutation A29D. In one or more embodiments, the patient has the mutation R38S. In one or more embodiments, the patient has the mutation N53Y. In one or more embodiments, the patient has the mutation Y88C. In one or more embodiments, the patient has the mutation V124G. In one or more embodiments, the patient has the mutation I133F. In one or more embodiments, the patient has the mutation A143V. In one or more embodiments, the patient has the mutation Y152N. In one or more embodiments, the patient has the mutation F159C. In one or more embodiments, the patient has the mutation A160D. In one or more embodiments, the patient has the mutation D165N. In one or more embodiments, the patient has the mutation F169I. In one or more embodiments, the patient has the mutation L180V. In one or more embodiments, the patient has the mutation D182G. In one or more embodiments, the patient has the mutation R196T. In one or more embodiments, the patient has the mutation W209R. In one or more embodiments, the patient has the mutation A257T. In one or more embodiments, the patient has the mutation P259S. In one or more embodiments, the patient has the mutation G271A. In one or more embodiments, the patient has the mutation S276T. In one or more embodiments, the patient has the mutation M290V. In one or more embodiments, the patient has the mutation A291S. In one or more embodiments, the patient has the mutation I303T. In one or more embodiments, the patient has the mutation I303V. In one or more embodiments, the patient has the mutation L310V. In one or more embodiments, the patient has the mutation G360A. In one or more embodiments, the patient has the mutation G360R. In one or more embodiments, the patient has the mutation G375A. In one or more embodiments, the patient has the mutation L394P. In one or more embodiments, the patient has the mutation G411S. In one or more embodiments, the patient has the mutation N419D. In various embodiments, these α-Gal A mutations are relative to the amino acid sequence shown in SEQ ID NO: 2.

Exemplary nucleotide changes associated with these novel mutations are shown in Table 1 below:

**Table 1: Novel Migalastat-Amenable Mutations**

| **Nucleotide change** | **Protein sequence change** |
|---|---|
| c.86C>A | A29D |
| c.114G>C | R38S |
| c.157A>T | N53Y |
| c.263A>G | Y88C |
| c.371T>G | V124G |
| c.397A>T | I133F |
| c.428C>T | A143V |
| c.454T>A | Y152N |
| c.476T>G | F159C |
| c.479C>A | A160D |
| c.493G>A | D165N |
| c.505T>A | F169I |
| c.538T>G | L180V |
| c.545A>G | D182G |
| c.587G>C | R196T |
| c.625T>A | W209R |
| c.769G>A | A257T |
| c.775C>T | P259S |
| c.812G>C | G271A |
| c.827G>C | S276T |
| c.868A>G | M290V |
| c.871G>T | A291S |
| c.908T>C | I303T |
| c.907A>G | I303V |
| c.928C>G | L310V |
| c. 1079G>C | G360A |
| c.1078G>C | G360R |
| c.1124G>C | G375A |
| c.1181T>C | L394P |
| c.1231G>A | G411S |
| c.1255A>G | N419D |

Accordingly, in various embodiments, migalastat is used to treat Fabry disease and/or enhance α-Gal A activity in a patient having a GLA mutation selected from the group consisting of: c.86C>A, c.114G>C, c.157A>T, c.263A>G, c.371T>G, c.397A>T, c.428C>T, c.454T>A, c.476T>G, c.479C>A, c.493G>A, c.505T>A, c.538T>G, c.545A>G, c.587G>C, c.625T>A, c.769G>A, c.775C>T, c.812G>C, c.827G>C, c.868A>G, c.871G>T, c.908T>C, c.907A>G, c.928C>G, c.1079G>C, c.1078G>C, c.1124G>C, c.1181T>C, c.1231G>A, and c.1255A>G. In one or more embodiments, the patient has the GLA mutation c.86C>A. In one or more embodiments, the patient has the GLA mutation c.114G>C. In one or more embodiments, the patient has the GLA mutation c.157A>T. In one or more embodiments, the patient has the GLA mutation c.263A>G. In one or more embodiments, the patient has the GLA mutation c.371T>G. In one or more embodiments, the patient has the GLA mutation c.397A>T. In one or more embodiments, the patient has the GLA mutation c.428C>T. In one or more embodiments, the patient has the GLA mutation c.454T>A. In one or more embodiments, the patient has the GLA mutation c.476T>G. In one or more embodiments, the patient has the GLA mutation c.479C>A. In one or more embodiments, the patient has the GLA mutation c.493G>A. In one or more embodiments, the patient has the GLA mutation c.505T>A. In one or more embodiments, the patient has the GLA mutation c.538T>G. In one or more embodiments, the patient has the GLA mutation c.545A>G. In one or more embodiments, the patient has the GLA mutation c.587G>C. In one or more embodiments, the patient has the GLA mutation c.625T>A. In one or more embodiments, the patient has the GLA mutation c.769G>A. In one or more embodiments, the patient has the GLA mutation c.775C>T. In one or more embodiments, the patient has the GLA mutation c.812G>C. In one or more embodiments, the patient has the GLA mutation c.827G>C. In one or more embodiments, the patient has the GLA mutation c.868A>G. In one or more embodiments, the patient has the GLA mutation c.871G>T. In one or more embodiments, the patient has the GLA mutation c.908T>C. In one or more embodiments, the patient has the GLA mutation c.907A>G. In one or more embodiments, the patient has the GLA mutation c.928C>G. In one or more embodiments, the patient has the GLA mutation c.1079G>C. In one or more embodiments, the patient has the GLA mutation c.1078G>C. In one or more embodiments, the patient has the GLA mutation c.1124G>C. In one or more embodiments, the patient has the GLA mutation c.1181T>C. In one or more embodiments, the patient has the GLA mutation c.1231G>A. In one or more embodiments, the patient has the GLA mutation c.1255A>G. In various embodiments, these GLA mutations are relative to the nucleic sequence shown in SEQ ID NO: 3.

Furthermore, various embodiments of the present invention provide PCs for the treatment of Fabry disease in a patient having a mutation in the gene encoding α-Gal A, wherein the patient is identified as having a missense mutation in a human α-Gal A encoded by a nucleic acid sequence set forth in SEQ ID NO: 1 and/or SEQ ID NO: 3. Another aspect of the invention pertains a method of treating a patient diagnosed with Fabry disease. In one or more embodiments, the method comprises administering to a patient a therapeutically effective dose of a PC of α-Gal A. In further embodiments, the patient has a missense mutation in the nucleic acid sequence encoding α-Gal A. Another aspect of the invention pertains to a method of enhancing α-Gal A in a patient diagnosed with or suspected of having Fabry disease. In one or more embodiments, the method comprises administering to a patient a therapeutically effective dose of a PC of α-Gal A, wherein the patient has a mutant α-Gal A encoded by a nucleic acid sequence having a missense mutation relative to SEQ ID NO: 1 and/or SEQ ID NO: 3. Details and further embodiments of these uses and methods follows below. Any of the embodiments relating a method of treating a patient with Fabry disease, a method of enhancing α-Gal A in a patient diagnosed with or suspected of having Fabry disease, use of a pharmacological chaperone for α-Gal A for the manufacture of a medicament for treating a patient diagnosed with Fabry disease or to a pharmacological chaperone for α-Gal A for use in treating a patient diagnosed with Fabry disease wherein the patient is identified as having a missense mutation in a human α-Gal A encoded by a nucleic acid sequence set forth in SEQ ID NO: 1 and/or SEQ ID NO: 3 can be combined with any of the other embodiments of the invention, for example embodiments relating to the PCs and suitable dosages thereof.

In one or more embodiments, the patient may have other mutations in their GLA gene. For example, there may be mutations in the intron region which may or may not affect the resulting α-Gal A enzyme. Thus, in one or more embodiments, the patient has mutant α-Gal A encoded by a nucleic acid sequence having at least 95, 96, 97, 98, 99, 99.1, 99.2, 99.3, 99.4, 99.5, 99.6, 99.7, 99.8 or 99.9% identity to SEQ ID NO: 1. Furthermore, the patient may have one or more additional mutations in the coding region of the GLA gene. Thus, in one or more embodiments, the patient has mutant α-Gal A encoded by a nucleic acid sequence having at least 95, 96, 97, 98, 99, 99.1, 99.2, 99.3, 99.4, 99.5, 99.6, 99.7, 99.8 or 99.9% identity to SEQ ID NO: 3. Moreover, in one or more embodiments, the patient has 1, 2, 3, 4, 5, 6, 7, 8, 9, 10, 15, 20 or 30 mutations relative to SEQ ID NO: 1 or SEQ ID NO: 3. It is also noted that some nucleic acid mutations in SEQ ID NO: 1 or SEQ ID NO: 3 can result in no change in amino acid for the resulting protein, as various amino acids are encoded by multiple nucleic acid sequences. Again, any of these embodiments can be combined with any of the other embodiments of the invention, for example embodiments relating to amenable mutations, the PCs and suitable dosages thereof.

### Pharmacological Chaperones

The binding of small molecule inhibitors of enzymes associated with LSDs can increase the stability of both mutant enzyme and the corresponding wild-type enzyme (see U.S. Pat. Nos. 6,274,597; 6,583,158; 6,589,964; 6,599,919; 6,916,829, and 7,141,582 all incorporated herein by reference). In particular, administration of small molecule derivatives of glucose and galactose, which are specific, selective competitive inhibitors for several target lysosomal enzymes, effectively increased the stability of the enzymes in cells *in vitro* and, thus, increased trafficking of the enzymes to the lysosome. Thus, by increasing the amount of enzyme in the lysosome, hydrolysis of the enzyme substrates is expected to increase. The original theory behind this strategy was as follows: since the mutant enzyme protein is unstable in the ER (Ishii et al., Biochem. Biophys. Res. Comm. 1996; 220: 812-815), the enzyme protein is retarded in the normal transport pathway (ER→Golgi apparatus -endosomes -lysosome) and prematurely degraded. Therefore, a compound which binds to and increases the stability of a mutant enzyme, may serve as a "chaperone" for the enzyme and increase the amount that can exit the ER and move to the lysosomes. In addition, because the folding and trafficking of some wild-type proteins is incomplete, with up to 70% of some wild-type proteins being degraded in some instances prior to reaching their final cellular location, the chaperones can be used to stabilize wild-type enzymes and increase the amount of enzyme which can exit the ER and be trafficked to lysosomes.

In one or more embodiments, the pharmacological chaperone comprises migalastat or a salt thereof. The compound migalastat, also known as 1-deoxygalactonojirimycin (1-DGJ) or (2R,3S,4R,5S)-2-(hydroxymethyl) piperdine-3,4,5-triol is a compound having the following chemical formula:

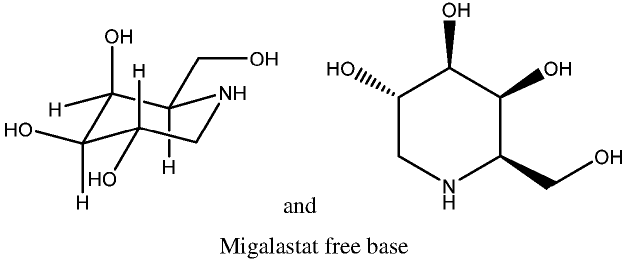

As discussed herein, pharmaceutically acceptable salts of migalastat may also be used in the present invention. When a salt of migalastat is used, the dosage of the salt will be adjusted so that the dose of migalastat received by the patient is equivalent to the amount which would have been received had the migalastat free base been used. One example of a pharmaceutically acceptable salt of migalastat is migalastat HCl:

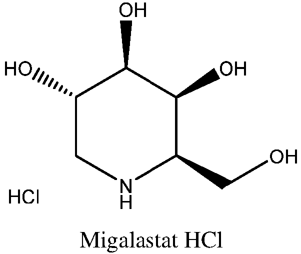

Migalastat is a low molecular weight iminosugar and is an analogue of the terminal galactose of GL-3. *In vitro* and *in vivo* pharmacologic studies have demonstrated that migalastat acts as a pharmacological chaperone, selectively and reversibly binding, with high affinity, to the active site of wild-type α-Gal A and specific mutant forms of α-Gal A. Migalastat binding stabilizes these mutant forms of α-Gal A in the endoplasmic reticulum facilitating their proper trafficking to lysosomes where dissociation of migalastat allows α-Gal A to reduce the level of GL-3 and other substrates.

In a specific embodiment, the PC comprises migalastat or salt thereof. In further embodiments, the PC comprises migalastat hydrochloride.

Any of these PCs for α-Gal A may be used in combination with any of the other embodiments of the invention, for example embodiments relating to a method of treating a patient with Fabry disease, a method of enhancing α-Gal A in a patient diagnosed with or suspected of having Fabry disease, use of a pharmacological chaperone for α-Gal A for the manufacture of a medicament for treating a patient diagnosed with Fabry disease or to a pharmacological chaperone for α-Gal A for use in treating a patient diagnosed with Fabry disease as well as embodiments relating to suitable doses of PCs, amenable mutations and to the treatment of a Fabry patient having certain mutations in the nucleic acid sequence encoding α-Gal A.

### Dosing, Formulation and Administration

In one or more embodiments, the Fabry patient is administered migalastat or salt thereof at a frequency of once every other day (also referred to as "QOD"). In various embodiments, the doses described herein pertain to migalastat hydrochloride or an equivalent dose of migalastat or a salt thereof other than the hydrochloride salt. In some embodiments, these doses pertain to the free base of migalastat. In alternate embodiments, these doses pertain to a salt of migalastat. In further embodiments, the salt of migalastat is migalastat hydrochloride. The administration of migalastat or a salt of migalastat is referred to herein as "migalastat therapy".

The effective amount of migalastat or salt thereof can be in the range from about 100 mg FBE to about 150 mg FBE. Exemplary doses include about 100 mg FBE, about 105 mg FBE, about 110 mg FBE, about 115 mg FBE, about 120 mg FBE, about 123 mg FBE, about 125 mg FBE, about 130 mg FBE, about 135 mg FBE, about 140 mg FBE, about 145 mg FBE or about 150 mg FBE.

Again, it is noted that 150 mg of migalastat hydrochloride is equivalent to 123 mg of the free base form of migalastat. Thus, in one or more embodiments, the dose is 150 mg of migalastat hydrochloride or an equivalent dose of migalastat or a salt thereof other than the hydrochloride salt, administered at a frequency of once every other day. As set forth above, this dose is referred to as 123 mg FBE of migalastat. In further embodiments, the dose is 150 mg of migalastat hydrochloride administered at a frequency of once every other day. In other embodiments, the dose is 123 mg of the migalastat free base administered at a frequency of once every other day.

In various embodiments, the effective amount is about 122 mg, about 128 mg, about 134 mg, about 140 mg, about 146 mg, about 150 mg, about 152 mg, about 159 mg, about 165 mg, about 171 mg, about 177 mg or about 183 mg of migalastat hydrochloride.

Accordingly, in various embodiments, migalastat therapy includes administering 123 mg FBE at a frequency of once every other day, such as 150 mg of migalastat hydrochloride every other day.

The administration of migalastat or salt thereof may be for a certain period of time. In one or more embodiments, the migalastat or salt thereof is administered for a duration of at least 28 days, such as at least 30, 60 or 90 days or at least 3, 4, 5, 6, 7, 8, 9, 10, 11, 12, 16, 20, 24, 30 or 36 months or at least 1, 2, 3, 4 or 5 years. In various embodiments, the migalastat therapy is long-term migalastat therapy of at least 6 months, such as at least 6, 7, 8, 9, 10, 11, 12, 16, 20, 24, 30 or 36 months or at least 1, 2, 3, 4 or 5 years.

Administration of migalastat or salt thereof according to the present invention may be in a formulation suitable for any route of administration, but is preferably administered in an oral dosage form such as a tablet, capsule or solution. As one example, the patient is orally administered capsules each containing 150 mg migalastat hydrochloride or an equivalent dose of migalastat or a salt thereof other than the hydrochloride salt.

In some embodiments, the PC *(e.g.,* migalastat or salt thereof) is administered orally. In one or more embodiments, the PC *(e.g.,* migalastat or salt thereof) is administered by injection. The PC may be accompanied by a pharmaceutically acceptable carrier, which may depend on the method of administration.

In one or more embodiments, the PC *(e.g.,* migalastat or salt thereof) is administered as monotherapy, and can be in a form suitable for any route of administration, including *e.g.,* orally in the form tablets or capsules or liquid, or in sterile aqueous solution for injection. In other embodiments, the PC is provided in a dry lyophilized powder to be added to the formulation of the replacement enzyme during or immediately after reconstitution to prevent enzyme aggregation *in vitro* prior to administration.

When the PC *(e.g.,* migalastat or salt thereof) is formulated for oral administration, the tablets or capsules can be prepared by conventional means with pharmaceutically acceptable excipients such as binding agents (e.g., pregelatinized maize starch, polyvinylpyrrolidone or hydroxypropyl methylcellulose); fillers *(e.g.,* lactose, microcrystalline cellulose or calcium hydrogen phosphate); lubricants (*e.g*., magnesium stearate, talc or silica); disintegrants *(e.g.,* potato starch or sodium starch glycolate); or wetting agents (*e.g*., sodium lauryl sulfate). The tablets may be coated by methods well known in the art. Liquid preparations for oral administration may take the form of, for example, solutions, syrups or suspensions, or they may be presented as a dry product for constitution with water or another suitable vehicle before use. Such liquid preparations may be prepared by conventional means with pharmaceutically acceptable additives such as suspending agents (*e.g*., sorbitol syrup, cellulose derivatives or hydrogenated edible fats); emulsifying agents *(e.g.,* lecithin or acacia); non-aqueous vehicles (*e.g*., almond oil, oily esters, ethyl alcohol or fractionated vegetable oils); and preservatives (*e.g*., methyl or propyl-p- hydroxybenzoates or sorbic acid). The preparations may also contain buffer salts, flavoring, coloring and sweetening agents as appropriate. Preparations for oral administration may be suitably formulated to give controlled release of the active chaperone compound.

The pharmaceutical formulations of the PC *(e.g.,* migalastat or salt thereof) suitable for parenteral/injectable use generally include sterile aqueous solutions (where water soluble), or dispersions and sterile powders for the extemporaneous preparation of sterile injectable solutions or dispersion. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be brought about by various antibacterial and antifungal agents, for example, parabens, chlorobutanol, phenol, benzyl alcohol, sorbic acid, and the like. In many cases, it will be reasonable to include isotonic agents, for example, sugars or sodium chloride. Prolonged absorption of the injectable compositions can be brought about by the use in the compositions of agents delaying absorption, for example, aluminum monosterate and gelatin.

Sterile injectable solutions are prepared by incorporating the purified enzyme (if any) and the PC *(e.g.,* migalastat or salt thereof) in the required amount in the appropriate solvent with various of the other ingredients enumerated above, as required, followed by filter or terminal sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

The formulation can contain an excipient. Pharmaceutically acceptable excipients which may be included in the formulation are buffers such as citrate buffer, phosphate buffer, acetate buffer, bicarbonate buffer, amino acids, urea, alcohols, ascorbic acid, and phospholipids; proteins, such as serum albumin, collagen, and gelatin; salts such as EDTA or EGTA, and sodium chloride; liposomes; polyvinylpyrollidone; sugars, such as dextran, mannitol, sorbitol, and glycerol; propylene glycol and polyethylene glycol *(e.g.,* PEG-4000, PEG-6000); glycerol; glycine or other amino acids; and lipids. Buffer systems for use with the formulations include citrate; acetate; bicarbonate; and phosphate buffers. Phosphate buffer is a preferred embodiment.

The route of administration of the chaperone compound may be oral or parenteral, including intravenous, subcutaneous, intra-arterial, intraperitoneal, ophthalmic, intramuscular, buccal, rectal, vaginal, intraorbital, intracerebral, intradermal, intracranial, intraspinal, intraventricular, intrathecal, intracisternal, intracapsular, intrapulmonary, intranasal, transmucosal, transdermal, or via inhalation.

Administration of the above-described parenteral formulations of the chaperone compound may be by periodic injections of a bolus of the preparation, or may be administered by intravenous or intraperitoneal administration from a reservoir which is external *(e.g.,* an i.v. bag) or internal *(e.g.,* a bioerodable implant).

Embodiments relating to pharmaceutical formulations and administration may be combined with any of the other embodiments of the invention, for example embodiments relating to methods of treating patients with Fabry disease, use of a pharmacological chaperone for α-Gal A for the manufacture of a medicament for treating a patient diagnosed with Fabry disease or to a pharmacological chaperone for α-Gal A for use in treating a patient diagnosed with Fabry disease as well as embodiments relating to amenable mutations, the PCs and suitable dosages thereof.

In one or more embodiments, the PC *(e.g.,* migalastat or salt thereof) is administered in combination with ERT. ERT increases the amount of protein by exogenously introducing wild-type or biologically functional enzyme by way of infusion. This therapy has been developed for many genetic disorders, including LSDs such as Fabry disease, as referenced above. After the infusion, the exogenous enzyme is expected to be taken up by tissues through non-specific or receptor-specific mechanism. In general, the uptake efficiency is not high, and the circulation time of the exogenous protein is short. In addition, the exogenous protein is unstable and subject to rapid intracellular degradation as well as having the potential for adverse immunological reactions with subsequent treatments. In one or more embodiments, the chaperone is administered at the same time as replacement enzyme (*e.g.,* replacement α-Gal A). In some embodiments, the chaperone is co-formulated with the replacement enzyme (*e.g*., replacement α-Gal A).

Reference throughout this specification to "one embodiment," "certain embodiments," "various embodiments," "one or more embodiments" or "an embodiment" means that a particular feature, structure, material, or characteristic described in connection with the embodiment is included in at least one embodiment of the invention. Thus, the appearances of the phrases such as "in one or more embodiments," "in certain embodiments," "in various embodiments," "in one embodiment" or "in an embodiment" in various places throughout this specification are not necessarily referring to the same embodiment of the invention. Furthermore, the particular features, structures, materials, or characteristics may be combined in any suitable manner in one or more embodiments.

Although the invention herein has been described with reference to particular embodiments, it is to be understood that these embodiments are merely illustrative of the principles and applications of the present invention. It will be apparent to those skilled in the art that various modifications and variations can be made to the method and apparatus of the present invention without departing from the spirit and scope of the invention. Thus, it is intended that the present invention include modifications and variations that are within the scope of the appended claims and their equivalents.

### EXAMPLE: Effect of Migalastat on Mutations of α-Gal A

The α-Gal A activity was measured in lysates prepared from HEK-293 cells transiently transfected with the indicated mutant form of α-Gal A and incubated in the absence or presence of 10 µM migalastat for 5 days. The α-Gal A activity is expressed as the nmoles of free 4-MU released per milligram of protein per hour (nmol/mg/hr). Baseline α-Gal A activity and α-Gal A activity after incubation with 10 µM migalastat, were additionally expressed as a percentage of baseline wild-type α-Gal A activity (% WT). The wild-type α-Gal A activity that was used to calculate these percentages was the average activity measured in lysates from wild-type transfected cells, incubated in the absence of migalastat, measured in parallel.

The results of the α-Gal A activity testing for the novel mutations A29D, R38S, N53Y, Y88C, V124G, I133F, A143V, Y152N, F159C, A160D, D165N, F169I, L180V, D182G, R196T, W209R, A257T, P259S, G271A, S276T, M290V, A291S, I303T, I303V, L310V, G360A, G360R, G375A, L394P, G411S, and N419D is shown in Table 2 below:

**Table 2: Effect of Migalastat on α-Gal A Activity**

| α-Gal A Mutant Form | Baseline α-Gal A activity (nmol/mg/hr) | 10 µM migalastat α-Gal A activity (nmol/mg/hr) | Mann-Whitney U p-value | Baseline α-Gal A activity (% WT) | 10 µM migalastat α-Gal A activity (% WT) | Absolute increase (% WT) | Relative increase |
|---|---|---|---|---|---|---|---|
| A29D | 5091 ± 413 | 6877 ± 474 | 0.0024 | 15.1 ± 1.1 | 20.3 ± 1.1 | 5.1 | 1.35 |
| R38S | 24042 ± 1962 | 30928 ± 1484 | 0.0026 | 73.3 ± 5.5 | 100 ± 9.3 | 26.6 | 1.29 |
| N53Y | 11238 + 1196 | 22871 ± 2192 | 0.0001 | 34 ± 2.1 | 68.5 ± 2.6 | 34.5 | 2.04 |
| Y88C | 1614 ± 64 | 5459 ± 307 | 0.0001 | 4.1 ± 0.2 | 14 ± 1.1 | 9.9 | 3.38 |
| V124G | 1750 ± 140 | 4316 ± 432 | 0.0001 | 4.3 ± 0.3 | 10.7 ± 1 | 6.4 | 2.47 |
| I133F | 293 ± 19 | 1542 ± 154 | 0.0001 | 0.7 ± 0.1 | 3.8 ± 0.3 | 3.1 | 5.27 |
| A143V | 4108 + 355 | 13309 ± 921 | 0.0001 | 13.7 ± 1 | 45.5 ± 3.2 | 31.7 | 3.24 |
| Y152N | 9417 ± 783 | 18554 ± 1297 | 0.0001 | 25.8 ± 1 | 51.6 ± 2.2 | 25.9 | 1.97 |
| F159C | 4472 ± 332 | 9238 ± 513 | 0.0001 | 13.8 + 0.9 | 29.6 ± 2.3 | 15.8 | 2.07 |
| A160D | 9985 ± 523 | 18238 ± 1048 | 0.0001 | 30.1 ± 1.7 | 56 ± 4.1 | 25.9 | 1.83 |
| D165N | 1540 ± 60 | 10562 ± 1017 | 0.0001 | 6 ± 0.3 | 40 ± 2.7 | 34 | 6.86 |
| F169I | 4622 ± 482 | 15903 ± 567 | 0.0001 | 15.9 + 2.1 | 51.7 ± 2.2 | 35.9 | 3.44 |
| L180V | 12900 ± 829 | 25437 ± 1279 | 0.0001 | 37.2 ± 2 | 73.4 ± 2.9 | 36.2 | 1.97 |
| D182G | 28037 ± 2162 | 43772 ± 4156 | 0.0014 | 84 ± 3.7 | 129.6 ± 9.7 | 45.6 | 1.56 |
| R196T | 17659 ± 1084 | 26217 ± 1611 | 0.0002 | 47 ± 2.1 | 69.5 ± 2.6 | 22.4 | 1.48 |
| W209R | 22944 ± 2196 | 28729 + 1293 | 0.0006 | 63.5 ± 5.4 | 81.1 ± 4.6 | 17.5 | 1.25 |
| A257T | 7405 ± 465 | 18990 ± 1196 | 0.0001 | 25.7 ± 1.5 | 66.2 ± 4.1 | 40.6 | 2.56 |
| P259S | 15425 ± 1009 | 28621 ± 3141 | 0.0001 | 44.3 ± 1.5 | 79.8 ± 4.8 | 35.5 | 1.86 |
| G271A | 4890 ± 570 | 18928 ± 1069 | 0.0001 | 12.1 ± 1.1 | 46.4 ± 1.8 | 34.4 | 3.87 |
| S276T | 547 ± 44 | 4104 ± 439 | 0.0001 | 1.3 ± 0.1 | 9.6 ± 0.9 | 8.3 | 7.5 |
| M290V | 23663 ± 2202 | 45804 ± 2927 | 0.0001 | 66.6 ± 6.2 | 128.1 ± 8.2 | 61.5 | 1.94 |
| A291S | 12908 ± 1151 | 20029 ± 1654 | 0.0036 | 47.7 ± 2.8 | 74.5 ± 4 | 26.8 | 1.55 |
| I303T | BLD | 2809 ± 520 | 0.0001 | BLD | 7.3 ± 1.4 | 7.3 | NC |
| I303V | 15157 ± 1669 | 28958 ± 3153 | 0.0001 | 38.2 ± 4.6 | 73.2 ± 8.8 | 35 | 1.91 |
| L310V | 23233 ± 3157 | 31936 ± 2527 | 0.0036 | 55.5 ± 6.3 | 78.4 ± 4.8 | 22.8 | 1.37 |
| G360A | 10510 ± 874 | 13443 ± 1048 | 0.0137 | 23.8 ± 1.4 | 31.3 ± 2.6 | 7.5 | 1.28 |
| G360R | 388 ± 48 | 1418 ± 93 | 0.0001 | 1.1 ± 0.1 | 4.0 ± 0.3 | 3 | 3.65 |
| G375A | 19691 ± 1167 | 24212 ± 1520 | 0.0169 | 54.8 ± 2.6 | 66.8 ± 2.4 | 12 | 1.23 |
| L394P | 5083 ± 465 | 7749 ± 582 | 0.0001 | 15.2 ± 2.1 | 22.3 ± 2.4 | 7.2 | 1.52 |
| G411S | 11132 ± 675 | 17543 ± 867 | 0.0001 | 39.1 ± 2.6 | 61.1 ± 3.2 | 22 | 1.58 |
| N419D | 10639 ± 512 | 14558 ± 678 | 0.0001 | 31.5 ± 1.5 | 44.1 ± 2.9 | 12.6 | 1.37 |

In Table 2, values for the mean ± standard error of the mean (SEM) were calculated. nmol/mg/hr indicates "nmoles of free 4-MU released per mg of protein per hour". WT indicates "wild-type". NC indicates "not calculable". N/A indicates "not applicable".

**Baseline and 10 µM migalastat α-Gal A activity:** Differences in the α-Gal A activity between lysates incubated in the absence and presence of 10 µM migalastat were determined using a one-tailed, Mann Whitney U test; an increase at 10 µM migalastat with a p<0.05 was considered significant. "BLD" indicates that the mean α-Gal A activity was below the limit of detection (<142 nmol/mg/hr).

**Baseline α-Gal A activity (% WT)** = (α-Gal A activity in mutant transfected cell lysates without migalastat ÷ α-Gal A activity in wild-type transfected cell lysates without migalastat) * 100.

**10 µM migalastat α-Gal A activity (% WT)** = (α-Gal A activity in mutant transfected cell lysates incubated with 10 µM migalastat ÷ α-Gal A activity in wild-type transfected cell lysates without migalastat) * 100.

**Absolute increase (% WT)** = is the 10 µM migalastat α-Gal A activity (% WT) minus the baseline α-Gal A activity (% WT).

**Relative increase** is the 10 µM migalastat α-Gal A activity in mutant transfected cell lysates ÷ baseline α-Gal A activity in mutant transfected cell lysates incubated without migalastat.

As can be seen from Table 2, the novel α-Gal A mutations A29D, R38S, N53Y, Y88C, V124G, I133F, A143V, Y152N, F159C, A160D, D165N, F169I, L180V, D182G, R196T, W209R, A257T, P259S, G271A, S276T, M290V, A291S, I303T, I303V, L310V, G360A, G360R, G375A, L394P, G411S, and N419D demonstrated an *in vitro* response to incubation with migalastat that met amenability criteria. Accordingly, patients with these mutations are expected to be treatable with migalastat therapy as described herein.

The patent and scientific literature referred to herein establishes the knowledge that is available to those with skill in the art. All United States patents and published or unpublished United States patent applications cited herein are incorporated by reference. All published foreign patents and patent applications cited herein are hereby incorporated by reference. All other published references, documents, manuscripts and scientific literature cited herein are hereby incorporated by reference.

While this invention has been particularly shown and described with references to preferred embodiments thereof, it will be understood by those skilled in the art that various changes in form and details may be made therein without departing from the scope of the invention encompassed by the appended claims.
The present invention is also exemplified by the following preferred embodiments:
1. A method for treatment of Fabry disease in a human patient in need thereof, the method comprising administering to the patient a therapeutically effective dose of migalastat or a salt thereof, wherein the patient has an α-galactosidase A mutation selected from the group consisting of: A29D, R38S, N53Y, Y88C, V124G, I133F, A143V, Y152N, F159C, A160D, D165N, F169I, L180V, D182G, R196T, W209R, A257T, P259S, G271A, S276T, M290V, A291S, I303T, I303V, L310V, G360A, G360R, G375A, L394P, G411S, and N419D.
2. The method of item 1, wherein the migalastat or salt thereof is administered to the patient every other day.
3. The method of item 1, wherein the patient is administered about 100 to about 150 mg free base equivalent of the migalastat or salt thereof every other day.
4. The method of item 1, wherein the patient is administered about 123 mg free base equivalent of the migalastat or salt thereof every other day.
5. The method of item 1, wherein the patient is administered about 123 mg of migalastat free base every other day.
6. The method of item 1, wherein the patient is administered about 150 mg of migalastat hydrochloride every other day.
7. The method of any one of items 1-6, wherein the migalastat or salt thereof is administered orally.
8. The method of any one of items 1-6, wherein the migalastat or salt thereof is administered by injection.
9. The method of any one of items 1-8, wherein the migalastat or salt thereof enhances α-galactosidase A activity.
10. The method of any one of items 1-9, wherein the patient is male.
11. The method of any one of items 1-9, wherein the patient is female.
12. The method of any one of items 1-11, wherein the mutation is A29D.
13. The method of any one of items 1-11, wherein the mutation is R38S.
14. The method of any one of items 1-11, wherein the mutation is N53Y.
15. The method of any one of items 1-11, wherein the mutation is Y88C.
16. The method of any one of items 1-11, wherein the mutation is V124G.
17. The method of any one of items 1-11, wherein the mutation is I133F.
18. The method of any one of items 1-11, wherein the mutation is A143V.
19. The method of any one of items 1-11, wherein the mutation is Y152N.
20. The method of any one of items 1-11, wherein the mutation is F159C.
21. The method of any one of items 1-11, wherein the mutation is A160D.
22. The method of any one of items 1-11, wherein the mutation is D165N.
23. The method of any one of items 1-11, wherein the mutation is F169I.
24. The method of any one of items 1-11, wherein the mutation is L180V.
25. The method of any one of items 1-11, wherein the mutation is D182G.
26. The method of any one of items 1-11, wherein the mutation is R196T.
27. The method of any one of items 1-11, wherein the mutation is W209R.
28. The method of any one of items 1-11, wherein the mutation is A257T.
29. The method of any one of items 1-11, wherein the mutation is P259S.
30. The method of any one of items 1-11, wherein the mutation is G271A.
31. The method of any one of items 1-11, wherein the mutation is S276T.
32. The method of any one of items 1-11, wherein the mutation is M290V.
33. The method of any one of items 1-11, wherein the mutation is A291S.
34. The method of any one of items 1-11, wherein the mutation is I303T.
35. The method of any one of items 1-11, wherein the mutation is I303V.
36. The method of any one of items 1-11, wherein the mutation is L310V.
37. The method of any one of items 1-11, wherein the mutation is G360A.
38. The method of any one of items 1-11, wherein the mutation is G360R.
39. The method of any one of items 1-11, wherein the mutation is G375A.
40. The method of any one of items 1-11, wherein the mutation is L394P.
41. The method of any one of items 1-11, wherein the mutation is G411S.
42. The method of any one of items 1-11, wherein the mutation is N419D.
43. The method of any one of items 1-42, wherein the mutation is disclosed in a pharmacological reference table.
44. The method of item 43, wherein the pharmacological reference table is provided in a product label for a migalastat product approved for the treatment of Fabry disease.
45. The method of item 43, wherein the pharmacological reference table is provided in a product label for GALAFOLD^{®}.
46. The method of item 43, wherein the pharmacological reference table is provided at a website.
47. The method of item 46, wherein the website is one or more of www.galafoldamenabilitytable.com or www.fabrygenevariantsearch.com.
48. A method of enhancing α-galactosidase A in a patient diagnosed with or suspected of having Fabry disease, the method comprising administering to the patient a therapeutically effective dose of migalastat or a salt thereof, wherein the patient has an α-galactosidase A mutation selected from the group consisting of:A29D, R38S, N53Y, Y88C, V124G, I133F, A143V, Y152N, F159C, A160D, D165N, F169I, L180V, D182G, R196T, W209R, A257T, P259S, G271A, S276T, M290V, A291S, I303T, I303V, L310V, G360A, G360R, G375A, L394P, G411S, and N419D.
49. The method of item 48, wherein the migalastat or salt thereof is administered to the patient every other day.
50. The method of item 48, wherein the patient is administered about 100 to about 150 mg free base equivalent of the migalastat or salt thereof every other day.
51. The method of item 48, wherein the patient is administered about 123 mg free base equivalent of the migalastat or salt thereof every other day.
52. The method of item 48, wherein the patient is administered about 123 mg of migalastat free base every other day.
53. The method of item 48, wherein the patient is administered about 150 mg of migalastat hydrochloride every other day.
54. The method of any one of items 48-53, wherein the migalastat or salt thereof is administered orally.
55. The method of any one of items 48-53, wherein the migalastat or salt thereof is administered by injection.
56. The method of any one of items 48-55, wherein the patient is male.
57. The method of any one of items 48-55, wherein the patient is female.
58. The method of any one of items 48-57, wherein the mutation is A29D.
59. The method of any one of items 48-57, wherein the mutation is R38S.
60. The method of any one of items 48-57, wherein the mutation is N53Y.
61. The method of any one of items 48-57, wherein the mutation is Y88C.
62. The method of any one of items 48-57, wherein the mutation is V124G.
63. The method of any one of items 48-57, wherein the mutation is I133F.
64. The method of any one of items 48-57, wherein the mutation is A143V.
65. The method of any one of items 48-57, wherein the mutation is Y152N.
66. The method of any one of items 48-57, wherein the mutation is F159C.
67. The method of any one of items 48-57, wherein the mutation is A160D.
68. The method of any one of items 48-57, wherein the mutation is D165N.
69. The method of any one of items 48-57, wherein the mutation is F169I.
70. The method of any one of items 48-57, wherein the mutation is L180V.
71. The method of any one of items 48-57, wherein the mutation is D182G.
72. The method of any one of items 48-57, wherein the mutation is R196T.
73. The method of any one of items 48-57, wherein the mutation is W209R.
74. The method of any one of items 48-57, wherein the mutation is A257T.
75. The method of any one of items 48-57, wherein the mutation is P259S.
76. The method of any one of items 48-57, wherein the mutation is G271A.
77. The method of any one of items 48-57, wherein the mutation is S276T.
78. The method of any one of items 48-57, wherein the mutation is M290V.
79. The method of any one of items 48-57, wherein the mutation is A291S.
80. The method of any one of items 48-57, wherein the mutation is I303T.
81. The method of any one of items 48-57, wherein the mutation is I303V.
82. The method of any one of items 48-57, wherein the mutation is L310V.
83. The method of any one of items 48-57, wherein the mutation is G360A.
84. The method of any one of items 48-57, wherein the mutation is G360R.
85. The method of any one of items 48-57, wherein the mutation is G375A.
86. The method of any one of items 48-57, wherein the mutation is L394P.
87. The method of any one of items 48-57, wherein the mutation is G411S.
88. The method of any one of items 48-57, wherein the mutation is N419D.
89. The method of any one of items 48-88, wherein the mutation is disclosed in a pharmacological reference table.
90. The method of item 89, wherein the pharmacological reference table is provided in a product label for a migalastat product approved for the treatment of Fabry disease.
91. The method of item 89, wherein the pharmacological reference table is provided in a product label for GALAFOLD^{®}.
92. The method of item 89, wherein the pharmacological reference table is provided at a website.
93. The method of item 92, wherein the website is one or more of www.galafoldamenabilitytable.com or www.fabrygenevariantsearch.com.

## Claims

1. Migalastat or a salt thereof for use in a method of enhancing α-galactosidase A in a patient diagnosed with or suspected of having Fabry disease, the method comprising administering to the patient a therapeutically effective dose of migalastat or a salt thereof, wherein the patient has an α-galactosidase A mutation selected from the group consisting of: A29D, R38S, N53Y, Y88C, V124G, 1133F, A143V, Y152N, F159C, A160D, D165N, F169I, L180V, D182G, R196T, W209R, A257T, P259S, G271A, S276T, M290V, A291S, I303T, 1303V, L310V, G360A, G360R, G375A, L394P, G411S, and N419D.

2. Migalastat or a salt thereof for use according to claim 1, wherein the migalastat or salt thereof is administered to the patient every other day.

3. Migalastat or a salt thereof for use according to claim 1, wherein the patient is administered about 100 to about 150 mg free base equivalent of the migalastat or salt thereof every other day.

4. Migalastat or a salt thereof for use according to claim 1, wherein the patient is administered about 123 mg free base equivalent of the migalastat or salt thereof every other day.

5. Migalastat or a salt thereof for use according to claim 1, wherein the patient is administered about 123 mg of migalastat free base every other day.

6. Migalastat or a salt thereof for use according to claim 1, wherein the patient is administered about 150 mg of migalastat hydrochloride every other day.

7. Migalastat or a salt thereof for use according to any one of claims 1-6, wherein the migalastat or salt thereof is administered orally.

8. Migalastat or a salt thereof for use according to any one of claims 1-6, wherein the migalastat or salt thereof is administered by injection.

9. Migalastat or a salt thereof for use according to any one of claims 1-8, wherein the patient is male.

10. Migalastat or a salt thereof for use according to any one of claims 1-8, wherein the patient is female.
